# EUROPEAN PATENT APPLICATION

(11) **EP 3 812 374 A1**
(43) Date of publication of application: **28.04.2021**
(21) Application number: 19204273.7
(22) Date of filing: 21.10.2019
(51) Int. Cl.: C07D 301/12

(54) **PROCESS FOR THE EPOXIDATION OF PROPENE**

(71) Applicant: Evonik Operations GmbH, 45128 Essen (DE); thyssenkrupp Industrial Solutions AG, 45143 Essen (DE)
(72) Inventor: KIBLER, Matthias, 64367 Mühltal (DE); DIETZ, Hans-Christian, 65795 Hattersheim (DE); HAJDUK, Martin, 63773 Goldbach (DE); WÖLL, Sigrid, 63477 Maintal (DE); WÖLL, Wolfgang, 63477 Maintal (DE); PICHA, Petra, 61381 Friedrichsdorf (DE); BREITENBACH, Uwe, 63584 Gründau (DE); SCHMIDT, Franz, 60386 Frankfurt (DE)
(74) Representative: Evonik Patent Association

(57) **Abstract**

In a process for continuously epoxidizing propene with hydrogen peroxide and an epoxidation catalyst, a liquid reaction mixture comprising dissolved propene is provided; a sample of this reaction mixture is withdrawn and its weight determined; the sample is depressurized providing a liquid sample and a vapor sample and the weight of one or both of these samples is determined; the content of at least one of hydrogen peroxide, oxygen, propene oxide and propene oxide byproducts is determined in of one or both of the samples; the content of a component in the reaction mixture is calculated from the content of the component in of one or both of the samples, the weight of the samples, and the weight of the sample of the reaction mixture; and a reaction parameter for reacting propene with hydrogen peroxide is adjusted based on the calculated content of a component in the reaction mixture.

## Description

### Field of the invention

The present invention relates to a continuous process for the epoxidation of propene with hydrogen peroxide in the presence of an epoxidation catalyst.

### Background of the invention

The epoxidation of propene with hydrogen peroxide in the presence of a titanium silicalite catalyst is known from EP 0 100 119 A1 and has found technical application in the so called HPPO process.

Epoxidation of propene with hydrogen peroxide is generally carried out with a molar excess of propene and at a pressure of more than 0.5 MPa in order to achieve a high reaction rate and a good propene oxide selectivity. When the epoxidation is carried out continuously with a heterogeneous catalyst, the catalyst will gradually lose catalytic activity and reaction conditions need to be adjusted to maintain a desired hydrogen peroxide conversion and propene oxide selectivity.

WO 01/10855 describes a continuous epoxidation of propene with hydrogen peroxide and a titanium silicalite catalyst where the reaction temperature and the pH of the hydrogen peroxide feed are adjusted to compensate changes in catalytic activity caused by catalyst deactivation. For this purpose, samples of the depressurized reaction mixture were analyzed by gas chromatography and hydrogen peroxide titration to determine hydrogen peroxide conversion and propene oxide selectivity. However, since the process of WO 01/10855 is operated with an excess of propene, the depressurization of the reaction mixture will vaporize a substantial part of the non-reacted propene. The concentrations determined for the depressurized reaction mixture will therefore be different from the concentrations of the original reaction mixture and calculating hydrogen peroxide conversion and propene oxide selectivity from concentrations determined for the depressurized reaction mixture will lead to erroneous values.

WO 02/063285 describes a method for a photometric online determination of hydrogen peroxide and its use for determining a hydrogen peroxide concentration in the reaction mixture of a continuous epoxidation of propene with hydrogen peroxide. For this purpose, a sample of 0.5-5 ml is sucked from a product stream through a capillary line to the sample vessel of an automated process titrator. However, when a sample of the epoxidation reaction mixture disclosed in WO 02/063285 is transferred by suction, vaporization of propene can occur with the effect that part of the transferred volume is occupied by vapor and not by liquid reaction mixture. Since the calculation of a hydrogen peroxide concentration of the reaction mixture carried out in WO 02/063285 is based on the assumption that the analyzed sample is all liquid, the vaporization of propene from the sample will lead to erroneous results.

Therefore, there is still a need for analyzing components of a reaction mixture of a continuous epoxidation of propene with hydrogen peroxide operated with a molar excess of propene, which provides correct values for the content of a component in the reaction mixture containing the non-reacted propene and is not affected by vaporization of propene from a reaction mixture sample.

### Summary of the invention

The inventors of the present invention have now found that analysis errors caused by vaporization of propene from the reaction mixture of a continuous epoxidation of propene with hydrogen peroxide operated with a molar excess of propene can be reliably avoided by weighing a sample before depressurizing it and weighing at least one of the liquid phase and the gas phase resulting from depressurization before analyzing one or both of the phases obtained by depressurization for a component, as this allows to calculate the correct content of the component in the original reaction mixture from the analysis data obtained from the depressurized sample. The calculated correct content of a component in the reaction mixture is then used to adjust a reaction parameter of the epoxidation reaction, providing a more precise control of operating conditions that is not affected by analysis errors.

Subject of the invention is therefore a process for the continuous epoxidation of propene with hydrogen peroxide, comprising
a) continuously reacting a molar excess of propene with hydrogen peroxide in the presence of an epoxidation catalyst to provide a liquid reaction mixture comprising dissolved propene at a propene partial pressure of from 0.1 to 5 MPa;
b) withdrawing a sample of said liquid reaction mixture and determining the weight of said sample;
c) depressurizing said sample to provide a liquid sample and a vapor sample and determining the weight of said liquid sample, said vapor sample or both;
d) determining the content of at least one component selected from hydrogen peroxide, oxygen, propene oxide and propene oxide byproducts in said liquid sample, said vapor sample or both;
e) calculating the content of a component in said liquid reaction mixture from the content of said component determined in step d), the weight of said liquid sample, said vapor sample or both determined in step c), and the weight of said sample of the liquid reaction mixture determined in step b); and
f) adjusting a reaction parameter for reacting propene with hydrogen peroxide in step a) based on the content of a component in said liquid reaction mixture calculated in step e).

### Detailed description of the invention

In step a) of the process of the invention, propene is continuously reacted with hydrogen peroxide in the presence of an epoxidation catalyst to provide a liquid reaction mixture comprising propene oxide and dissolved propene at a propene partial pressure of from 0.1 to 5 MPa.

Propene is used in a molar excess to hydrogen peroxide, preferably at a molar ratio of propene to hydrogen peroxide of from 1.1:1 to 30:1, more preferably 2:1 to 10:1 and most preferably 3:1 to 5:1. In a preferred embodiment, propene is used in an excess sufficient to maintain an additional liquid phase rich in propene throughout step a). The propene may contain propane, preferably with a molar ratio of propane to propene of from 0.001 to 0.15 and more preferably of from 0.08 to 0.12.

Hydrogen peroxide can be used as an aqueous solution, preferably containing from 30 to 75 % by weight hydrogen peroxide and most preferably from 40 to 70 % by weight. The aqueous hydrogen peroxide solution is preferably made by an anthraquinone process.

The reaction of propene with hydrogen peroxide can be carried out in the absence or in the presence of a solvent and is preferably carried out in the presence of a solvent. Suitable are all solvents which are not oxidized or are oxidized to only a small extent by hydrogen peroxide under the reaction conditions chosen and which dissolve in water in an amount of more than 10 % by weight. Solvents which are completely miscible with water are preferred. Particularly suitable solvents are alcohols, such as methanol, ethanol or tert-butanol; glycols, such as ethylene glycol, 1,2-propanediol or 1,3-propanediol; cyclic ethers, such tetrahydrofuran, dioxane or propylene oxide; glycol ethers, such ethylene glycol monomethyl ether, ethylene glycol monoethyl ether, ethylene glycol monobutyl ether or the propylene glycol monomethyl ethers; ketones, such as acetone or 2-butanone; and nitriles, such as acetonitrile and propionitrile. The propene is preferably reacted with hydrogen peroxide in a methanol solvent. The methanol solvent can be a technical grade methanol, a solvent stream recovered in the work-up of the epoxidation reaction mixture or a mixture of both. The methanol solvent may comprise other solvents in minor amounts, such as ethanol, with the amount of such other solvents preferably being less than 2 % by weight. The methanol solvent may also comprise water, preferably from 2 to 13 % by weight water. The solvent is preferably used in the epoxidation in a weight ratio of 0.5 to 20 relative to the combined weight of water and hydrogen peroxide.

The epoxidation catalyst used in step a) preferably comprises a titanium zeolite containing titanium atoms on silicon lattice positions. Preferably, a titanium silicalite catalyst is used, preferably with an MFI or MEL crystal structure. Most preferably a titanium silicalite-1 catalyst with MFI structure as known from EP 0 100 119 A1, is used. The titanium silicalite catalyst is preferably employed as a shaped catalyst in the form of granules, extrudates or shaped bodies. For the shaping process the catalyst may contain 1 to 99% of a binder or carrier material, all binders and carrier materials being suitable that do not react with hydrogen peroxide or with propene oxide under the reaction conditions employed for the epoxidation, silica being preferred as binder. Extrudates with a diameter of 1 to 5 mm are preferably used as shaped catalysts. The amount of catalyst employed may be varied within wide limits and is preferably chosen so that a hydrogen peroxide consumption of more than 90%, preferably more than 95%, is achieved within 1 minute to 5 hours under the employed epoxidation reaction conditions.

The epoxidation reaction of step a) is preferably carried out at a temperature of 20 to 80°C, more preferably at 25 to 60°C. The epoxidation reaction is preferably carried out at a pressure that is higher than the vapor pressure of propene at the reaction temperature in order to maintain the propene dissolved in the solvent or present as a separate liquid phase. The pressure in step a) is preferably from 1.9 to 5.0 MPa, more preferably 2.1 to 3.6 MPa and most preferably 2.4 to 2.8 MPa. Using an excess of propene at a high pressure provides high reaction rate and hydrogen peroxide conversion and at the same time high selectivity for propene oxide.

The epoxidation reaction is preferably carried out with addition of an additive modifying the selectivity of said epoxidation catalyst. When the epoxidation catalyst comprises a titanium zeolite, a base is preferably used as additive for modifying the selectivity of said epoxidation catalyst. Most preferably, the epoxidation catalyst comprises a titanium silicalite and ammonia is used as a base to improve epoxide selectivity as described in EP 0 230 949 A2. Ammonia is preferably added with a weight ratio of ammonia to the initial amount of hydrogen peroxide of from 0.0001 to 0.003.

The epoxidation reaction of step a) is preferably carried out in a fixed bed reactor by passing a mixture comprising propene, hydrogen peroxide and solvent over a fixed bed comprising a shaped titanium zeolite catalyst. The fixed bed reactor is preferably a tube bundle reactor and the catalyst fixed bed is arranged inside the reactor tubes. The fixed bed reactor is preferably equipped with cooling means and cooled with a liquid cooling medium. The temperature profile along the length of the catalyst fixed bed is preferably adjusted to keep the reaction temperature along 70 to 98 %, preferably along 80 to 95 %, of the length of the catalyst fixed bed within a range of less than 5 °C, preferably within a range of from 0.5 to 3 °C. The temperature of the cooling medium fed to the cooling means is preferably adjusted to a value 3 to 13 °C lower than the maximum temperature in the catalyst fixed bed. The epoxidation reaction mixture is preferably passed through the catalyst bed in down flow mode, preferably with a superficial velocity from 1 to 100 m/h, more preferably 5 to 50 m/h, most preferred 5 to 30 m/h. The superficial velocity is defined as the ratio of volume flow rate/cross section of the catalyst bed. Additionally it is preferred to pass the reaction mixture through the catalyst bed with a liquid hourly space velocity (LHSV) from 1 to 20 h⁻¹, preferably 1.3 to 15 h⁻¹. It is particularly preferred to maintain the catalyst bed in a trickle bed state during the epoxidation reaction. Suitable conditions for maintaining the trickle bed state during the epoxidation reaction are disclosed in WO 02/085873 on page 8 line 23 to page 9 line 15. The epoxidation reaction is most preferably carried out with a catalyst fixed bed maintained in a trickle bed state at a pressure close to the vapor pressure of propene at the reaction temperature, using an excess of propene that provides a reaction mixture comprising two liquid phases, a solvent rich phase and a propene rich liquid phase. Two or more fixed bed reactors may be operated in parallel or in series in order to be able to operate the epoxidation process continuously when regenerating the epoxidation catalyst.

In step b) of the process of the invention, a sample of the liquid reaction mixture provided in step a) is withdrawn and the weight of the sample is determined. The weight of the sample is preferably determined by weighing the container receiving the sample before and after withdrawing the sample.

In a preferred embodiment, the sample is withdrawn at a higher pressure than the pressure used in step a) for carrying out the epoxidation reaction in order to prevent evaporation of components during withdrawal of the sample. In another preferred embodiment, the sample is withdrawn with cooling, preferably cooling the sample by 2 to 80 °C in order to prevent evaporation of components during withdrawal of the sample. The liquid reaction mixture provided in step a) is preferably withdrawn with cooling to a temperature of from 5 to 30 °C cooling the sample by at least 5 °C. Withdrawing the sample in step b) without evaporation of components during withdrawal prevents fractionation of volatile components resulting from vapor entering the sample container formed by flashing from liquid which does not enter the sample container.

In step c) of the process of the invention, the sample withdrawn in step b) is depressurized to provide a liquid sample and a vapor sample and the weight of the resulting liquid sample, the resulting vapor sample or both samples is determined. The weight of the liquid sample can be determined by weighing a container before and after receiving the liquid sample. The weight of the vapor sample can be determined by connecting a container containing the sample withdrawn in step b) with an evacuated vessel, depressurizing the sample of step b) passing the resulting vapor into this evacuated vessel and weighing the vessel before and after receiving the vapor resulting from depressurization.

In a preferred embodiment of the process of the invention, steps b) and c) are carried out continuously and the weights of the continuously obtained samples are determined as mass flow rates. All types of mass flow meters known from the prior art can be used for this embodiment.

Depressurizing in step c) is preferably carried out at a temperature of from 0 to 80 °C, more preferably at a temperature of from 40 to 60 °C. When a solvent is used in step a), depressurizing is preferably carried out at a temperature which is from 5 to 40 °C lower than the boiling point of the solvent.

In step d) of the process of the invention, the content of at least one component is determined for the liquid sample or the vapor sample obtained in step c) or for both the liquid sample and the vapor sample. The component may be hydrogen peroxide and the content of hydrogen peroxide is determined for the liquid sample. The component may be oxygen and the content of oxygen is determined for the vapor sample. The component may be propene oxide and the content of propene oxide is determined for both the liquid sample and the vapor sample. The component may be a propene oxide byproduct, such as 1,2-propanediol or a propylene glycol monomethyl ether if methanol is used as solvent in step a), and the content of the propene oxide byproduct is determined for the liquid sample. Step d) may also involve determining any combination of these components.

The content of hydrogen peroxide in the liquid sample can be determined by methods known from the prior art. The content of hydrogen peroxide may be determined by redox titration, preferably by redox titration with an oxidant oxidizing hydrogen peroxide to oxygen, such as titration with permanganate orcerimetric titration with a soluble Ce(IV) salt, such as Ce(IV) sulfate. Alternatively, the content of hydrogen peroxide may be determined by reacting the hydrogen peroxide with a reagent, such as titanyl sulfate, to give a colored reaction product and photometrically determining the content of the colored reaction product. Suitable reagents and methods for photometrically determining hydrogen peroxide content are known from the prior art, such as from WO 02/063285 page 9, line 4 to page 11, line 8. amperometric sensor is used for determining the concentration of hydrogen peroxide, particularly The content of hydrogen peroxide may also be determined with an amperometric sensor by electrochemical oxidation of hydrogen peroxide according to the reaction equation

H₂O₂ → O₂ + 2 H⁺ + 2 e⁻.

Suitable amperometric sensors for hydrogen peroxide are commercially available, for example from ProMinent® under the name DULCOTEST® PEROX. In a further alternative, the content of hydrogen peroxide is determined by Raman spectroscopy, preferably as described in WO 2016/131649 on page 6, lines 23 to 33.

The content of oxygen in the vapor sample can also be determined by methods known from the prior art, such as by gas chromatography, by Raman spectroscopy or by measuring magnetic susceptibility.

The content of propene oxide in the liquid sample and the vapor sample can be determined by known methods, such as gas chromatography and Raman spectroscopy, and is preferably determined by capillary gas chromatography. A part of the liquid sample may be treated with a reductant to reduce hydrogen peroxide and other peroxides before analyzing it by gas chromatography in order to prevent oxidative aging of the GC separation column.

The content of propene oxide byproducts in the liquid sample can also be determined by gas chromatography and is preferably determined along with the content of propene oxide in the same GC analysis. When a methanol solvent is used in step a), the contents of propene oxide byproducts 1-methoxy-2-propanol and 2-methoxy-1-propanol are preferably determined in step d).

In step e) of the process of the invention, the content of a component in the liquid reaction mixture provided in step a) is calculated from the content of this component as determined in step d), from the weight of the liquid sample, the vapor sample or both as determined in step c), and the weight of the sample of the liquid reaction mixture as determined in step b). The content of hydrogen peroxide in the liquid reaction mixture can be calculated by multiplying the content of hydrogen peroxide determined in the liquid sample provided in step c) with the ratio of the weight of the liquid sample provided in step c) to the weight of the sample withdrawn in step b), because the vapor sample provided in step b) contains essentially no hydrogen peroxide. When steps b) and c) are carried out continuously, this calculation can be made with the ratio of the corresponding mass flows. The content of oxygen in the liquid reaction mixture can be calculated with sufficient precision by multiplying the content of oxygen determined in the vapor sample provided in step c) with the ratio of the weight of the vapor sample provided in step c) to the weight of the sample withdrawn in step b), because the liquid sample provided by depressurizing in step b) has only a low content of dissolved oxygen. The content of propene oxide in the liquid reaction mixture can be calculated by multiplying the content of propene oxide determined in the liquid sample provided in step c) with the ratio of the weight of the liquid sample provided in step c) to the weight of the sample withdrawn in step b) and adding to this value the value resulting from multiplying the content of propene oxide determined in the vapor sample provided in step c) with the ratio of the weight of the vapor sample provided in step c) to the weight of the sample withdrawn in step b). If only the weight of the liquid sample is determined in step c), the weight of the vapor sample may be calculated as the difference between the weight of the sample withdrawn in step b) and the weight of the liquid sample provided in step c).

In step f) of the process of the invention, a reaction parameter for reacting propene with hydrogen peroxide in step a) is adjusted based on the content of a component in the liquid reaction mixture provided in step a) as calculated in step e). The reaction parameter which is adjusted in step f) is preferably selected from the reaction temperature at which propene is reacted with hydrogen peroxide, the feed rate for hydrogen peroxide, and the feed rate of an additive which modifies the selectivity of the epoxidation catalyst used in step a).

In a first preferred embodiment of the invention, in step d) the content of hydrogen peroxide is determined in the liquid sample provided in step c); in step e) the content of hydrogen peroxide is calculated from the content of hydrogen peroxide determined in step d), the weight of the liquid sample determined in step c) and the weight of the sample of the liquid reaction mixture determined in step b); and in step f) the reaction temperature for reacting propene with hydrogen peroxide in step a) is adjusted to maintain an essentially constant hydrogen peroxide conversion. Essentially constant here means that the variation in hydrogen peroxide conversion, averaged over a day and excluding start-up of the epoxidation reaction and any interruption of the reaction, does not exceed a range of +/- 2 %. The reaction temperature can be adjusted by cooling the reactor with a liquid coolant and adjusting the coolant temperature, the coolant flow or both. The content of hydrogen peroxide in the liquid sample can be determined in step d) by the methods described further above and is preferably determined by redox titration with an oxidant, most preferably by cerimetric titration or titration with permanganate. The content of hydrogen peroxide in the liquid reaction mixture is preferably calculated in step e) as described further above for step e).

In a second preferred embodiment of the invention, in step d) the content of at least one propene oxide byproduct is determined in the liquid sample provided in step c); in step e) the content of said propene oxide byproduct is calculated from the content of the propene oxide byproduct determined in step d), the weight of the liquid sample determined in step c) and the weight of the sample of the liquid reaction mixture determined in step b); and in step f) the feed rate of an additive modifying the selectivity of the epoxidation catalyst is adjusted to maintain an essentially constant propene oxide selectivity. Essentially constant here means that the variation in propene oxide selectivity, averaged over a day and excluding start-up of the epoxidation reaction and any interruption of the reaction, does not exceed a range of +/- 2 %. Propene oxide selectivity is defined for this purpose as the ratio of the content of propene oxide in the liquid reaction mixture to the total content of propene oxide and determined propene oxide byproducts in the liquid reaction mixture. Preferably, the content of propene oxide is determined in step d) in the liquid sample and the vapor sample; in step e) the content of propene oxide is calculated from the contents of propene oxide determined in step d), the weight of the liquid sample determined in step c) and the weight of the sample of the liquid reaction mixture determined in step b); and the propene oxide selectivity is calculated from the contents of propene oxide and at least one propene oxide byproduct calculated in step e).

Suitable additives for modifying the selectivity of the epoxidation catalyst are known from the prior art. In a further preferred embodiment, the epoxidation catalyst comprises a titanium zeolite and a base is used as additive modifying the selectivity of the epoxidation catalyst. More preferably, the epoxidation catalyst comprises titanium silicalite and ammonia is used as the base for modifying the selectivity of the epoxidation catalyst. Ammonia is preferably added in an amount of from 250 to 2000 ppm based on the mass flow of hydrogen peroxide to step a).

In a third preferred embodiment of the invention, the epoxidation catalyst comprises a titanium zeolite; in step d) the content of oxygen is determined in the vapor sample; in step e) the content of oxygen is calculated from the content of oxygen determined in step d), the weight determined in step c) and the weight of the sample of the liquid reaction mixture determined in step b); and in step f) regeneration of the catalyst is initiated when the molar ratio of molecular oxygen formed in step a) to the hydrogen peroxide fed to step a) exceeds a threshold value. The amount of molecular oxygen formed in step a) can be calculated from the content of oxygen in the liquid reaction mixture determined in step e). The threshold value is preferably set to a value in the range of from 0.01 to 0.1, more preferably to a value in the range of from 0.04 to 0.08.

The titanium zeolite catalyst can be regenerated by calcination, by treatment with a heated gas, preferably an oxygen containing gas, by treatment with an oxidant in the absence of propene, preferably with hydrogen peroxide, or by a solvent wash. The titanium zeolite catalyst is preferably regenerated by washing it with a solvent, preferably at a temperature of from 80 to 250 °C, more preferably at a temperature of from 100 to 200 °C. The solvent used for regenerating the titanium zeolite catalyst is preferably selected from alcohols, glycols, cyclic ethers, glycol ethers, ketones, nitriles and combinations thereof and preferably comprises methanol, most preferably in an amount of from 50 to 100 % by weight. Regeneration with a methanol solvent is preferably carried out as described in WO 2005/000827. When a solvent is used in step a), the titanium zeolite catalyst is preferably regenerated by washing it with the same solvent as used in step a). When a fixed bed reactor is used in step a) of the process, the regeneration of the titanium zeolite catalyst is preferably carried out without removing it from the fixed bed reactor.

Different methods of regeneration may be combined. Preferably, the catalyst is regenerated by washing with a solvent until the catalyst activity obtained after regeneration has dropped below a desired level. The catalyst is then regenerated by calcination or by treatment with a heated gas, preferably by thermal treatment at 80 to 600 °C, more preferably at 250 to 550 °C, with a gas stream containing oxygen.

### Examples

A continuous epoxidation of propene was carried out in a test reactor using a reaction tube with a cooling jacket. The reaction tube had an internal diameter of 30.5 mm and contained a catalyst fixed bed of 10 I of an extruded titanium silicalite catalyst. A mixture of 40 % by weight of propene, 7.7 % by weight of hydrogen peroxide, 3.3 % by weight of water, 49 % by weight of methanol and 77 ppm by weight ammonia was fed to the top of the reaction tube and passed through the catalyst fixed bed in trickle mode. The pressure in the reactor was maintained at 2.6 MPa by introducing nitrogen and the temperature in the reactor was adjusted by controlling coolant flow to maintain an essentially constant conversion of hydrogen peroxide of 97 to 98 %, compensating activity loss of the catalyst by increasing the reaction temperature. The liquid reaction mixture leaving the reaction tube was depressurized to ambient pressure. The liquid phase remaining after depressurizing was analyzed for propene oxide by GC and for hydrogen peroxide by cerimetric redox titration and propene oxide yield and hydrogen peroxide conversion were calculated from the amount of hydrogen peroxide fed and the contents of propene oxide and hydrogen peroxide in the liquid reaction mixture leaving the reaction tube, which in turn were calculated from the analysis results, the weight of a sample of the liquid reaction mixture leaving the reaction tube, and the weight of the liquid phase remaining after depressurizing.

Table 1 summarizes the hydrogen peroxide conversion and the changes in the average temperature in the catalyst fixed bed and in the propene oxide yield over the course of the epoxidation reaction.

**Table 1**

| Total run time in h | Hydrogen peroxide conversion in % | Propene oxide yield on H₂O₂ in % | Average temperature in catalyst fixed bed in °C |
|---|---|---|---|
| 45 | 97,0 | 83,1 | 28,8 |
| 104 | 97,9 | 83,7 | 32,7 |
| 152 | 98,0 | 85,8 | 34,2 |
| 201 | 97,5 | 86,4 | 34,5 |
| 249 | 97,4 | 86,2 | 35,4 |
| 297 | 97,7 | 86,1 | 36,4 |
| 357 | 98,1 | 86,1 | 38,0 |
| 405 | 98,0 | 86,0 | 37,9 |
| 453 | 97,7 | 86,3 | 38,0 |
| 501 | 97,5 | 84,0 | 38,5 |
| 597 | 97,8 | 85,4 | 40,0 |
| 705 | 97,6 | 84,4 | 41,2 |
| 801 | 97,7 | 83,7 | 42,0 |
| 897 | 98,1 | 84,4 | 43,3 |
| 1002 | 97,5 | 82,9 | 42,8 |
| 1098 | 97,7 | 84,2 | 44,5 |
| 1206 | 97,5 | 83,3 | 45,3 |
| 1303 | 97,7 | 83,3 | 46,5 |
| 1399 | 97,7 | 83,0 | 47,7 |
| 1495 | 97,8 | 82,1 | 48,6 |
| 1603 | 97,5 | 81,5 | 49,3 |
| 1699 | 97,8 | 81,8 | 50,8 |
| 1795 | 97,7 | 81,0 | 51,5 |
| 1903 | 97,5 | 80,7 | 52,1 |
| 1999 | 98,3 | 80,4 | 55,4 |

## Claims

1. A process for the continuous epoxidation of propene with hydrogen peroxide, comprising
a) continuously reacting a molar excess of propene with hydrogen peroxide in the presence of an epoxidation catalyst to provide a liquid reaction mixture comprising dissolved propene at a propene partial pressure of from 0.1 to 5 MPa;
b) withdrawing a sample of said liquid reaction mixture and determining the weight of said sample;
c) depressurizing said sample to provide a liquid sample and a vapor sample and determining the weight of said liquid sample, said vapor sample or both;
d) determining a content of hydrogen peroxide in said liquid sample, a content of oxygen in said vapor sample, a content of propene oxide in both said liquid sample and said vapor sample, a content of a propene oxide byproduct in said liquid sample, or any combination of said contents;
e) calculating the content of a component in said liquid reaction mixture from the content of said component determined in step d), the weight of said liquid sample, said vapor sample or both determined in step c), and the weight of said sample of the liquid reaction mixture determined in step b); and
f) adjusting a reaction parameter for reacting propene with hydrogen peroxide in step a) based on the content of a component in said liquid reaction mixture calculated in step e).

2. The process of claim 1, wherein the reaction parameter is selected from the reaction temperature, the feed rate for hydrogen peroxide, and the feed rate of an additive modifying the selectivity of said epoxidation catalyst.

3. The process of claim 1 or 2, wherein in step d) the content of hydrogen peroxide is determined in the liquid sample; in step e) the content of hydrogen peroxide is calculated from the content of hydrogen peroxide determined in step d), the weight of the liquid sample determined in step c) and the weight of the sample of the liquid reaction mixture determined in step b); and in step f) the reaction temperature for reacting propene with hydrogen peroxide is adjusted to maintain an essentially constant hydrogen peroxide conversion.

4. The process of claim 3, wherein in step d) the content of hydrogen peroxide in the liquid sample is determined by redox titration with an oxidant, preferably by cerimetric titration or titration with permanganate.

5. The process of claim 1 or 2, wherein in step d) the content of at least one propene oxide byproduct is determined in the liquid sample; in step e) the content of said propene oxide byproduct is calculated from the content of said propene oxide byproduct determined in step d), the weight of the liquid sample determined in step c) and the weight of the sample of the liquid reaction mixture determined in step b); and in step f) the feed rate of an additive modifying the selectivity of said epoxidation catalyst is adjusted to maintain an essentially constant propene oxide selectivity.

6. The process of claim 5, wherein in step d) the content of propene oxide is determined in the liquid sample and the vapor sample; in step e) the content of propene oxide is calculated from the contents of propene oxide determined in step d), the weight of the liquid sample determined in step c) and the weight of the sample of the liquid reaction mixture determined in step b); and the propene oxide selectivity is calculated from the contents of propene oxide and at least one propene oxide byproduct calculated in step e).

7. The process of claim 5 or 6, wherein a methanol solvent is used in step a) and contents of propene oxide byproducts 1-methoxy-2-propanol and 2-methoxy-1-propanol are determined in step d).

8. The process of any one of claims 5 to 7, wherein the epoxidation catalyst comprises a titanium zeolite and a base is used as additive modifying the selectivity of the epoxidation catalyst.

9. The process of claim 8, wherein the epoxidation catalyst comprises titanium silicalite and ammonia is used as the base.

10. The process of claim 1 or 2, wherein the epoxidation catalyst comprises a titanium zeolite; in step d) the content of oxygen is determined in the vapor sample; in step e) the content of oxygen is calculated from the content of oxygen determined in step d), the weight determined in step c) and the weight of the sample of the liquid reaction mixture determined in step b); and in step f) regeneration of the catalyst is initiated when the molar ratio of molecular oxygen formed in step a) to the hydrogen peroxide fed to step a) exceeds a threshold value.

11. The process of claim 10, wherein depressurizing in step c) is carried out at a temperature of from 0 to 80 °C, preferably from 40 to 60 °C.

12. The process of any one of claims 1 to 11, wherein steps b) and c) are carried out continuously and the weights of the continuously obtained samples are determined as mass flow rates.

13. The process of any one of claims 1 to 12, wherein in step b) the sample is withdrawn with cooling.

14. The process of claim 13, wherein the sample is cooled by 2 to 80 °C.
